# EUROPEAN PATENT SPECIFICATION

(11) **EP 2 736 369 B1**
(45) Date of publication and mention of the grant of the patent: **14.02.2018**
(21) Application number: 12817537.9
(22) Date of filing: 24.07.2012
(51) Int. Cl.: A61F 2/00, A61F 13/00, A61B 17/064, A61B 17/12, A61B 17/068, A61B 17/32, A61B 17/122, A61B 17/128, A61B 17/00

(54) **SYSTEM FOR CONNECTING TISSUE PATCHES**
SYSTEM ZUM VERBINDEN VON GEWEBE-PATCHES
SYSTÈME DE LIAISON DE PATCHS TISSULAIRES

(30) Priority: 26.07.2011 US 201161511720 P
(43) Date of publication of application: 04.06.2014
(73) Proprietor: Covidien LP, Mansfield, MA 02048 (US)
(72) Inventor: MARCZYK, Stanislaw, Stratford, Connecticut 06614 (US)
(74) Representative: Soames, Candida Jane
(86) International application number: PCT/US2012/047930
(87) International publication number: WO 2013/016316

(56) References cited:
- EP-A1- 2 229 918
- KR-B1- 100 982 628
- US-A1- 2002 116 070
- US-A1- 2004 220 591
- US-A1- 2009 204 129
- US-A1- 2010 256 674
- US-A1- 2011 144 417

## Description

### CROSS-REFERENCE TO RELATED APPLICATION

### BACKGROUND

### Technical Field

The present disclosure relates to the mesh patches for the correction of tissue defects. More particularly, the present disclosure relates to systems and methods for joining two or more sections of mesh material to form a mesh patch suitable for a particular procedure.

### Background of Related Art

Incisional, ventral, epigastric and umbilical hernias are defects of the anterior abdominal wall. These defect may be congenital, i.e., umbilical hernia, or acquired, i.e., incisional. Incisional hernias form after surgery and are formed through the incision site, through previous drain sites, and/or through laparoscopic trocar insertion sites.

While surgical repair of inguinal or femoral hernias may only require application of smaller prosthetic mesh patches, repair of incisional, ventral, epigastric or umbilical hernias may require application of larger mesh patches. Obese patients, patients having week abdominal walls or patients with large hernia defects may also need larger mesh patches to cover not only the hernia defect but also the margins of the defect. The use of a patch having a size sufficient to cover a defect and the margins of the defect has been shown to reduce the reoccurrence rate of the defect.

During laparoscopic hernia repair, the desire for minimal scarring limits the size of the opening through which the surgical site is accessed. The size of the opening limits the size of the mesh patch that may be inserted through the opening. In addition, once received through the opening, manipulating a larger patch within the body cavity may also present issues. US 2011/144417 A1 discloses an implantable patch made of a plurality of patches having a loop structure. The connection between the patches can be done by clips.

Therefore, it would be beneficial to have a system and method for inserting smaller patches and connecting the smaller patches to form a larger mesh patch.

### SUMMARY

A system for connecting mesh laproscopically is provided according to the features of claim 1. The system includes a first patch, a second patch and a connection means for joining the first and second patches. Each of the first and second patches includes a base and a plurality of loops extending along at least a portion of a periphery of the base. The connection means is configured for maintaining at least a first loop of the first plurality of loops adjacent at least a first loop of the second plurality of loops.

The connection means, according to the invention is a first clip including a body defining a cavity and an extension secured to the body, the extension being pivotable within the cavity to permit receipt of the first loops of the first and second plurality of loops within the cavity. The connection means may include a staple, a clip or a wire. The connection means may instead include a plurality of hook members formed on the first loop of the first plurality of loops and a plurality of loop members formed on the first loop of the second plurality of loops for engaging the hook members. In one embodiment, the system may further include a clip applier for applying a clip to the first loops of the first and second plurality of loops. Alternatively, the system may include a stapling device for forming a staple about the first loops of the first and second plurality of loops. In yet another embodiment, the system includes an applier for applying a wire about the first loops of the first and second plurality of loops. In still another embodiment, the system includes a welding device for fusing at least the first loop of the first and second plurality of loops together.

Also provided is a method of laparoscopically joining two or more patches. The method includes the step of providing first and second patches within a body cavity, wherein each of the patches includes at least one loop formed along a periphery thereof. The method further includes the steps of positioning at least one loop of the first patch adjacent at least one loop of the second patch and applying a connection means about the at least one loop of the first patch and the at least one loop of the second patch to secure the first patch with the second patch. The method may further include the step of applying additional connection means about other adjacent loops. The connecting means may include a staple, a clip or a wire.

Another method of laparoscopically joining two or more patches is provided. The method includes the step of providing first and second patches, wherein each of the patches includes at least one loop formed along a periphery thereof. The method further includes the steps of positioning the at least one loop of the first patch adjacent the at least one loop of the second patch and applying a heat source to the at least one loop of the first patch and the at least one loop of the second patch to fuse the loops together.

Still another method of laparoscopically joining two or more patches is provided. The method include the step of providing first and second patches, wherein each of the patches includes at least one loop formed along a periphery thereof, the at least one loop of the first patch including a plurality of hook members formed thereon and the at least one loop of the second patch including a plurality of loop members formed thereon. The method further includes the steps of positioning the at least one loop of the first patch adjacent the at least on loop of the second patch and contacting the at least one loop of the first patch into engagement with the at least one loop of the second patch such that the hook members and the loop members engage.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a top view of a mesh patch according to an embodiment of the present disclosure;
FIG. 2 is a top view of first and second patches according to the embodiment of FIG. 1;
FIG. 3 is a perspective view of the first and second patches of FIG. 2 and connection means connecting the first and second patches;
FIG. 4 is a sectional view of the first and second patches of FIG. 2, as the patches are connected by a stapling device;
FIG. 5 is the sectional view of the first and second patches of FIG. 2 connected by first and second embodiments of staples according to the present disclosure;
FIG. 5A is a side view of the first embodiment of the staple of FIG. 5;
FIG. 5B is a side view of the second embodiment of the staple of FIG. 5;
FIG. 6 is the sectional view of the first and second patches of FIG. 2 connected by a third embodiment of a staple according to the present disclosure;
FIG. 6A is a side view of the third embodiment of the staple of FIG. 6;
FIG. 7A is a perspective view of an embodiment of a clip according to the present disclosure;
FIG. 7B is a perspective view of the clip of FIG. 7A, connecting a pair of loops;
FIG. 8 is a clip applier for applying the clip of FIGS. 7A and 7B;
FIG. 9 is a perspective view of another embodiment of a clip according to the present disclosure;
FIG. 10 is a perspective view of yet another embodiment of a clip according to the present disclosure;
FIG. 11 is a perspective view of a first loop connected to a second loop with a wire;
FIG. 12 is a perspective view of an applier for applying the wire of FIG. 11;
FIG. 13 is a perspective view of a welding device according to the present disclosure for fusing together first and second loops; and
FIG. 14 is a perspective sectional view of a first patch having loop members formed on a loop and a second patch having hook members formed on a loop.

### DETAILED DESCRIPTION

Although the systems and methods of the present disclosure will be described as relates to a laparoscopic procedures, it is envisioned that the present disclosed systems and methods may be used and performed endoscopcially, arthroscopically, through an incision or through a naturally occurring orifice, i.e., anus or vagina.

The present disclosure relates to apparatus, systems and methods for joining two or more smaller patches to form a larger mesh patch. Preferably the two or more smaller patches are connected once received within a body cavity or once positioned adjacent a target site, however, it is envisioned that the two or more sections of material may be joined external of the body. Although the present disclosure will relate to the material of the sections to be joined as being formed of mesh, the sections of material may include non-mesh configurations. For example, the section of material may include a sheet of fabric, polymer or other suitable structure for providing support to a tissue defect.

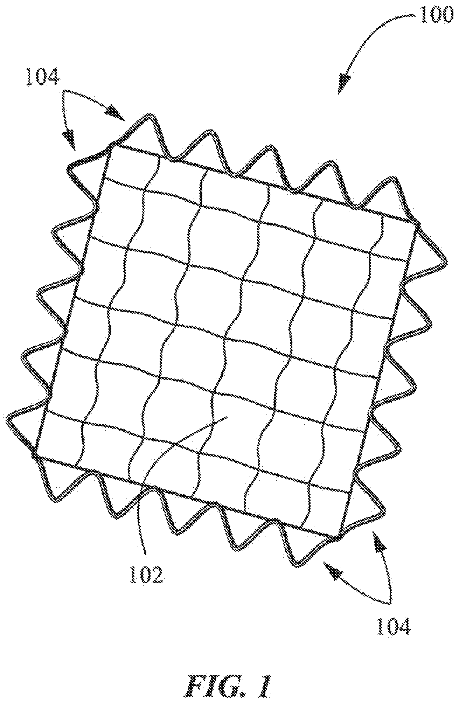

Referring initially to FIG. 1, a section of material according to the present disclosure is shown generally as patch 100. Patch 100 includes a base 102 and a plurality of loops 104 extending about a periphery of base 102. As discussed above, base 102 may be formed of mesh or any other suitable material for providing support to a tissue defect or repair. Base 102 may be absorbable or non-absorbable. As shown, base 102 includes a substantially rectangular shape, however, it is envisioned that base 102 may be provided in any shape, e.g., triangular, square, circular, star, cross. Loops 104 extend about the periphery of base 102 and may be formed of the same or different material as base 102. In one embodiment, loops 104 are integrally formed with base 102. Alternatively, loops 104 may be welded, adhered, glued or otherwise bonded to base 102. Loops 104 may be of any size and/or any configuration. The number and/or size of loops 104 may vary depending on the type of tissue at the target site and/or the type of material forming base 102. As shown, loops 104 extend about the entire periphery of base 102. In an alternative embodiment, loops 104 may only extend about a portion of the periphery of base 102.

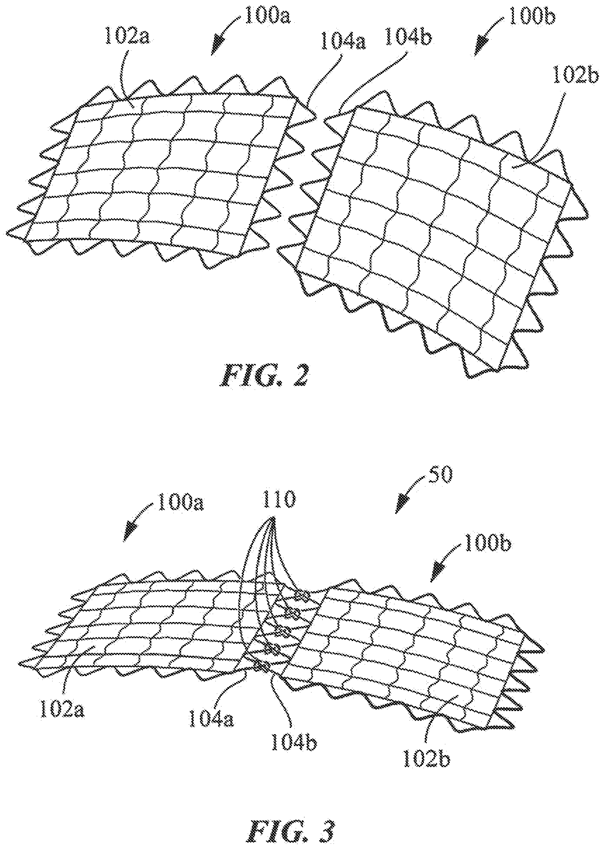

With reference to FIG. 2, joining a first patch 100a with a second patch 100b requires positioning first patch 100a relative to second patch 100b such that at least one of loops 104a on first patch 100a is adjacent at least one of loops 104b on second patch 100b. As discussed above, first and second patches 100a, 100b are preferably joined within a body cavity, i.e., in proximity to the target site during a laparoscopic procedure, however, first and second patches 100a, 100b may be joined prior to insertion within the body. Patches 100a, 100b may be inserted within the body cavity using any number of methods and/or devices. As is known in the art, first and second patches 100a, 100b may be rolled into an elongated tubular structure (not shown) and inserted through an opening into the body cavity. Alternatively, first and second patches 100a, 100b may be inserted through the opening with the use of one or more insertion devices.

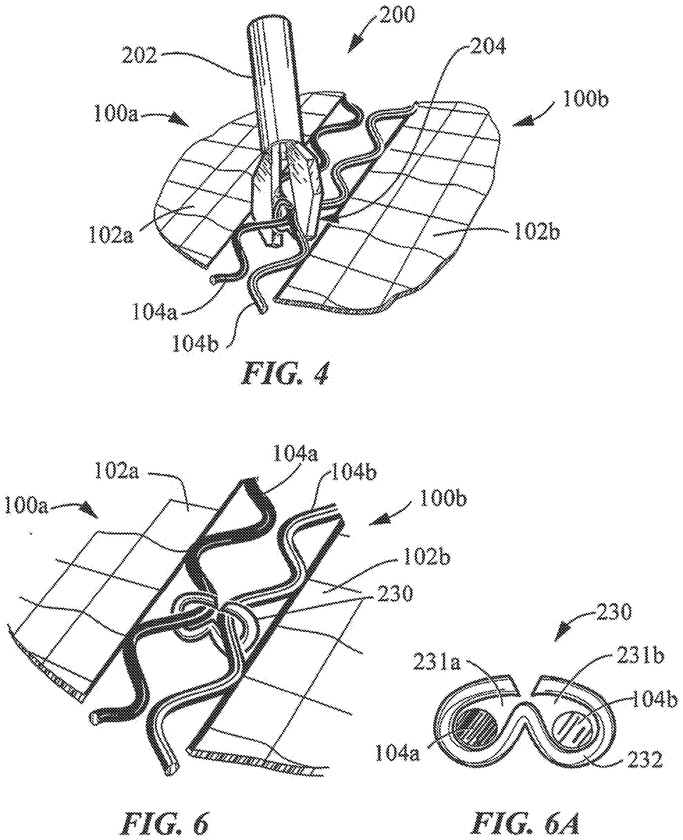

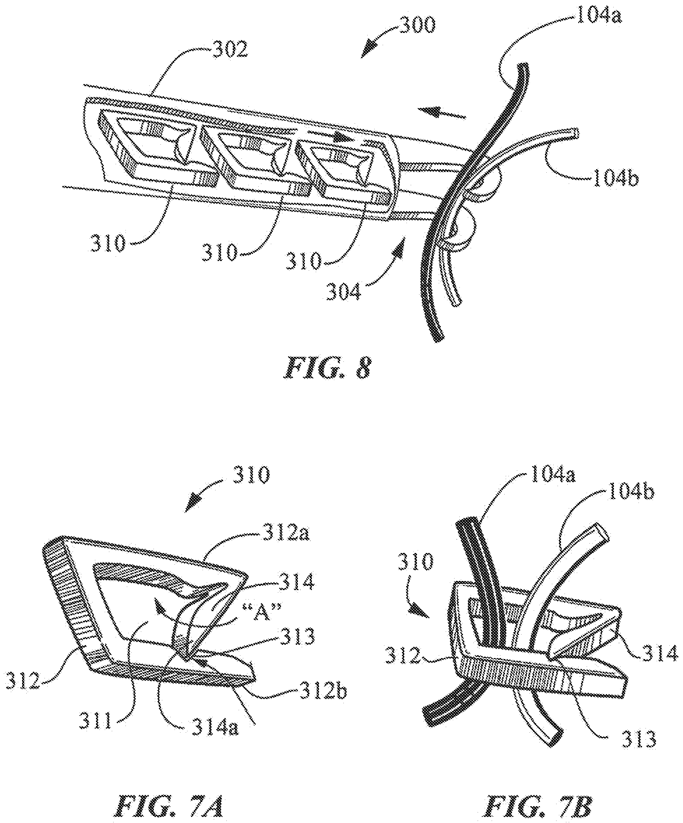

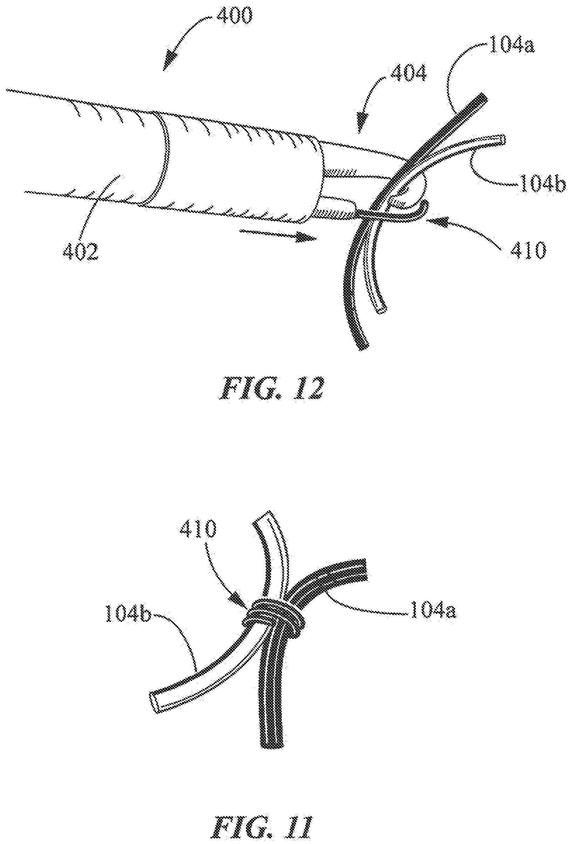

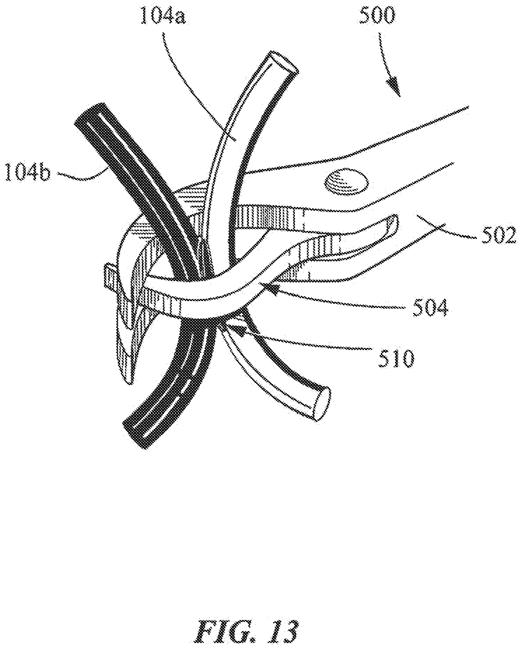

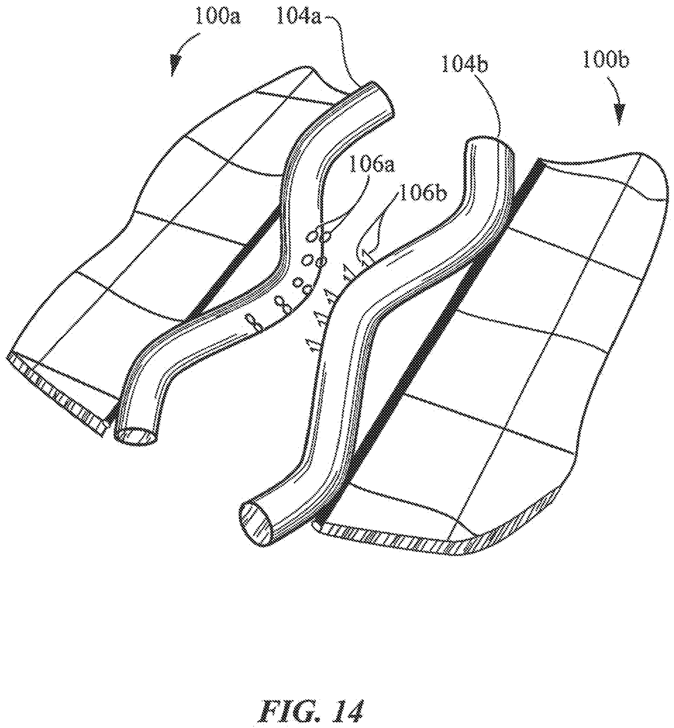

Referring now to FIG. 3, once first and second patches 100a, 100b are positioned adjacent one another, one or more connection means 110 are used to secure first patch 100a with second patch 100b. More particularly, connection means 110 secures loops 104a of first patch 100a with loops 104b of second patch 100b. Connection means 110 may include staples (FIGS. 4-6B), clips (FIGS. 7A-10), wire (FIGS. 11 and 12), welded/fused section (FIG. 13), hook and loop fasteners (FIG. 14) and/or other connectors known in the art. Connection means 110 may be applied to each set of loops 104a, 104b. Alternatively, connection means 110 may be applied to every other set of loops 104a, 104b, every third set of loops 104a, 104b, or in any other suitable configuration. Although first and second patches 100a, 100b are shown joined prior to securing either of first or second patches 100a, 100b to tissue, it is envisioned that one or both of first and second patches 100a, 100b may be secured to tissue before first and second patches 100a, 100b are joined together. Although the following discussion relates to the joining of first and second patches 100a, 100b, it is envisioned that the apparatus and methods of the present disclosure may be used to join more than two patches. If is further envisioned that more than one type of connection means 110 may be used to connect first and second patches 100a, 100b.

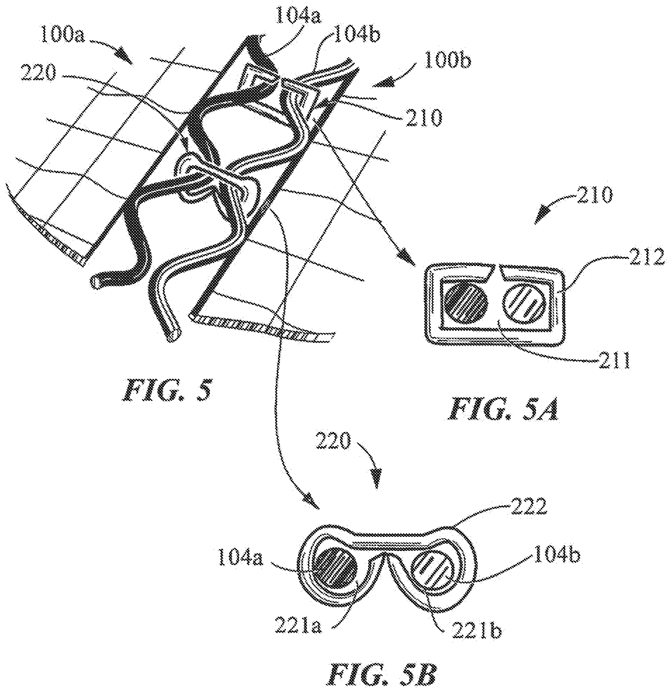

With reference to FIGS. 4-6B, in one method of connecting first and second patches 100a, 100b, the connection means joining loops 104a of first patch 100a with loops 104b of second patch 100b includes a plurality of staples. The staples for joining first and second patches 100a, 100b may include existing or modified skin and fascia staples (FIGS. 5-6A), existing or known ligating staples (FIGS. 6 and 6A) or any other suitable staple.

With reference initially to FIG. 4, a device for securing a staple about loops 104a, 104b of first and second patches 100a, 100b is shown generally as stapling device 200. Although referred to as stapling device 200, the device for applying staples may also be referred to as a clip applier. Stapling device 200 includes an elongated body 202 and j aw members 204. Stapling device 200 is configured to engage loops 104a, 104b of respective first and second patches 100a, 100b and to form a staple about loops 104a, 104b. As discussed above, since first and second patches 100a, 100b are preferably joined within a body cavity during a laparoscopic procedure, elongated body 202 and jaw members 204 may be configured for laparoscopic use. Thus, elongated body 202 and jaw members 204 are configured for insertion through an incision, access port or other suitable opening.

With reference still to FIG. 4, stapling device 200 may be any one of a number of known staplers for applying a staple to skin and fascia. For example, as seen in FIGS. 5-5B, skin and fascia staples 210, 220 are used to connect loop 104a of first patch 100a with loop 104b of second patch 100b. With particular reference to FIG. 5A, when formed, staple 210 includes a body 212 having a box-like configuration defining a substantially rectangular recess 211. Body 212 of staple 210 is configured such that, upon staple formation, loops 104a, 104b of respective first and second patches 100a, 100b are received and secured within cavity 211. First and second ends 212a, 212b of body 212 may be pointed as shown, or may instead be rounded, flat or otherwise configured to prevent damage to first and second patches 100a, 100b and surrounding tissue. As shown, the configuration of body 212 is such that pointed ends 212a, 212b of body 212 are directed towards one another so as to prevent injury to first and second patches 100a, 100b and surrounding tissue.

With particular reference now to FIG. 5B, when formed, staple 220 includes a body 222 having a substantially hour-glass configuration defining a pair of cavities 221a, 221b. Cavities 221a, 221b are each configured to receive a loop 104a, 104b of first and second patches 100a, 100b, respectively. As with staple 210, described above, ends 222a, 222b of body 222 may be pointed, as shown, rounded, flat or otherwise configured to prevent damage to first and second patches 100a, 100b and surrounding tissue.

With reference back to FIGS. 4 and 5, stapling device 200 may be modified to form one or both of staples 210, 220. In use, loops 104a, 104b of respective first and second patches 100a, 100b are received within jaw members 204 of stapling device 200. Actuation of stapling device 200 causes the deformation of staple 210, 220 about loops 104a, 104b. As discussed above, staple 210 includes cavity 211 for receiving loops 104a, 104b. Thus, as staple 210 is formed, loops 104a, 104b are simultaneously received within cavity 211. Staple 220 may be deformed in a similar manner. Alternatively, first and second cavities 221a, 221b of staple 220 may be individually formed. In this manner, body 222 of staple 220 may be deformed about loop 104a to secure loop 104a within cavity 221a and then body 222 may be deformed about loop 104b to secure loop 104b within cavity 221b. As such, second patch 100b may be positioned adjacent first patch 100a subsequent to receiving loop 104a of first patch 100a within cavity 221a, thereby permitting manipulation of second patch 100b relative to first patch 100a during the connecting of first and second patches 100a, 100b.

With reference to FIGS. 6 and 6A, in another embodiment, staple 230 includes a body 232 having a substantially C-shaped configuration defining a pair of cavities 231a, 231b. As with staple 220 described hereinabove, staple 230 is configured to receive loop 104a of first patch 100a in a first cavity 231a and loop 104b of second patch 100b in a second cavity 231b. Similarly to staple 220, loops 104a, 104b may be received within respective cavities 231a, 231b individually or simultaneously.

With reference now to FIGS. 7A-10, in another method of connecting first and second patches 100a, 100b, the connection means joining first and second patches 100a, 100b includes one or more clips. It is envisioned that each of the below described clips may be selectively disengageable such that first and second patches 100a, 100b may be disconnected. In this manner the first and second patches 100a, 100b may be more easily removed or reconfigured.

Referring initially to FIGS. 7A and 7B, clip 310 is configured for securing loops 104a of first patch 100a (FIG. 2) with loops 104b of second patch 100b. Clip 310 includes a body 312 having a substantially C-shape configuration defining a substantially rectangular cavity 311. A hinged extension 314 extends from a first end 312a of body 312. Hinged extension 314 is biased outwardly and is configured to pivot within cavity 311, as indicated by arrow "A" (FIG. 7A). A notch 313 is formed in a second end 312b of body 312 and is configured to selectively receive a free end 314a of extension 314 and to maintain extension 314 within cavity 311.

In use, and as seen in FIG. 7B, when loops 104a, 104b of first and second patches 100a, 100b, respectively, are received within cavity 311, receipt of free end 314a of extension 314 within notch 313 secures loops 104a, 104b within cavity 311, thereby connecting first and second patches 100a, 100b. Multiple clips 310 may be applied to loops 104a, 104b along the length of patches 100a, 100b, respectively, to further secure first and second patches 100a, 100b.

Clip 310 may be applied using forceps, tweezers and/or any other suitable device or devices. Turning to FIG. 8, in one embodiment, a clip applier 300 is used for applying clips 310 to connect first and second patches 100a, 100b. Clip applier 300 includes an elongated body 302 and jaw members 304. Elongated body 302 of clip applier 300 is configured to retain and selectively dispense a plurality of clips 310. Jaw members 304 of clip applier 300 are configured to selectively engage loops 104a, 104b of respective first and second patches 100a, 100b. Activation of clip applier 300 causes loops 104a, 104b to come into contact with extension 314, thereby pivoting extension 314 inwardly and permitting reception of loops 104a, 104b within cavity 311 of body 312. Loops 104a, 104b of first and second patches 100a, 100b, respectively, are received within cavity 311 of body 312 when clip 310 is advanced distally towards jaw members 314. Alternatively, retraction of jaw members 304 relative to elongated body 302 of clip applier 300 causes engagement of loops 104a, 104b with extension 314 and subsequent reception of loops 104a, 104b within cavity 311. In another embodiment, jaw members 304 may be retracted as clip 310 is advanced.

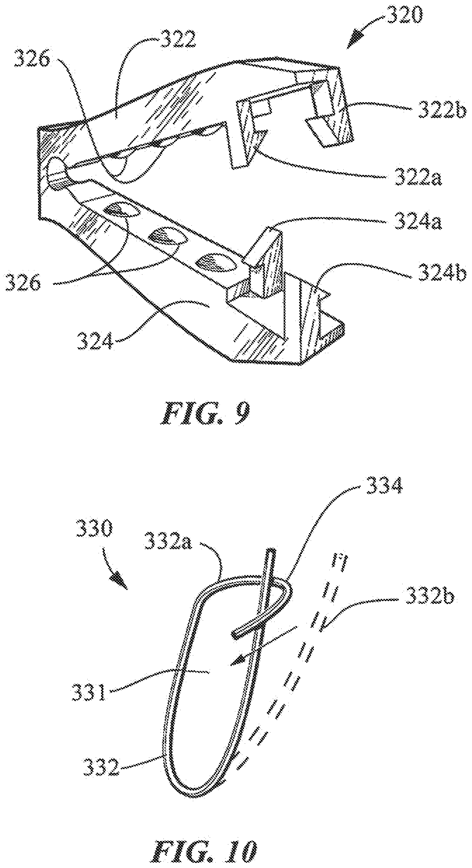

With reference now to FIG. 9, in an alternative example, clip 320 is configured to receive and retain loops 104a, 104b of respective first and second patches 100a, 100b. Clip 320 includes a first arm 322 hingedly connected to a second arm 324. Each of first and second arms 322, 324 include a pair of inwardly facing tabs 322a, 322b, 324a, 324b. Each of tabs 322a, 322b of first arm 322 correspond with tabs 324a, 324b of second arm 324. Approximation of either or both of first arm 322 or second arm 322 towards the other arm causes engagement of tabs 322a, 324a, 322b, 324b. Tabs 322a, 322b, 324a, 324b are configured such that upon engagement with each other first and second arms 322, 324 are locked relative to each other. A gap or space 321 formed between first and second arms 322, 324 is configured to receive loops 104a, 104b of respective first and second patches 100a, 100b. Either or both of first and second arms 322, 324 may include raised portion 326 or otherwise be configured to more securely retain loops 104a, 104b of first and second patches 100a, 100b, respectively. Clip 320 may be applied with forceps, tweezers or any other suitable device. In the event clip 320 is applied to first and second patches 100a, 100b external the body cavity or during an open procedure, clip 320 may be applied by hand.

Turning now to FIG. 10, in another embodiment, clip 330 includes a body 332 having a substantially U-shaped configuration defining a cavity 331. A first end 332a of body 332 includes a catch portion 334 configured to receive a second end 332b of body 332. Clip 330 is configured such that, upon reception of loops 104a, 104b of first and second patches 100a, 100b within cavity 331, second end 332b of body 332 may be approximated towards first end 332a of body 332 such that second end 332b engages catch portion 334, thereby retaining loops 104a, 104b within cavity 331. As with clip 320, clip 330 may be applied to first and second patches 100a, 100b using forceps, tweezers, a clip applier or any other suitable device. In the event clip 330 is applied to first and second patches 100a, 100b external the body cavity or during an open procedure, clip 330 may be applied by hand.

With reference now to FIGS. 11 and 12, in another method of connecting first and second patches 100a, 100b, the connection means joining first and second patches 100a, 100b includes one or more wires. Referring initially to FIG. 11, wire 410 is wrapped about loops 104a, 104b of respective first and second patches 100a, 100b. Wire 410 may be formed of an absorbable or non-absorbable metal, polymer or other suitable material. In one embodiment, wire 410 is formed of a shape memory material that is configured to assume a coiled shape upon release of a restraining force or upon achievement of another condition. In one embodiment, wire 410 may be formed of nitinol.

Turning now to FIG. 12, wire 410 may be wrapped about loops 104a, 104b of first and second patches 100a, 100b, respectively, with the assistance of an applier 400. Applier 400 includes an elongated body 402 and a jaw member 404. Jaw member 404 is configured to selectively engage loops 104a, 104b while elongated body 402 is configured to selectively eject wire 410 therefrom. Jaw member 404 may include an anvil (not shown) for forming wire 410 into a coiled configuration for reception about loops 104a, 104b. Wire 410 may be provided in preselected lengths, or may instead be formed of a continuous length and require cutting to size. In this manner, applier 400 may further include a cutting mechanism.

Alternatively, wire 410 is wrapped about loops 104a, 104b using forceps, tweezers, graspers or any other suitable device. In the event first and second patches 100a, 100b are joined external of the body cavity or during an open procedure, wire 410 may be applied about loops 104a, 104b by hand.

With reference now to FIG. 13, in still another method of connecting first and second patches 100a, 100b, loops 104a, 104b of respective first and second patches 100a, 100b are fused together. As shown, a welding device 500, including an elongated body 502 and jaw members 504, is configured to fuse together loops 104a, 104b. Jaw members 504 may be operably connect to a heater, ultrasonic horn or other suitable device for selectively heating and melting adjacent portions of loops 104a, 104b. Jaw members 504 are further configured to approximate loops 104a, 104b towards one another to facilitate the fusing of loops 104a, 104b with each other. It is envisioned that loops 104a, 104b may include a coating to facilitate joining of loops 104a, 104b. Welding device 500 is preferably configured for laparoscopic use, however, welding device 500 may be configured for use in open procedures.

Turning now to FIG. 14, in yet another method of connecting first and second patches 100a, 100b, loop 104a of first patch 100a includes loop members 106a and loop 104b of second patch 104b includes hook members 106b. Loop members 106a may be formed along one or more of loops 104a. Hook members 106b may be formed along one or more of loops 104b. Loop members 106a of loop 104a are configured to engage hook members 106b of loop 104b. Engagement of loop members 106a of loop 104a of first patch 100a with hook members 106b of loop 104b of second patch 100b secures loop 104a to loop 104b, thereby connecting first and second patches 100a, 100b. In this manner, loops 104a, 104b may be secured together by hand, using forceps, pliers or any other suitable method for squeezing loops 104a, 104b together.

Once formed, large patch 50 (FIG. 3) may be used as a traditional tissue patch. As discussed above, it is envisioned that one or both of first and second patches 100a, 100b may be secured to tissue prior to first and second patches 100a, 100b being connected.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure.

## Claims

1. A system for connecting patches (100) laparoscopically, the system comprising:
a first patch (100a) including a first base (102a) and a first plurality of loops (104a) extending along at least a portion of a lateral edge of the first base;
a second patch (100b) including a second base (102b) and a second plurality of loops (104b) extending along at least a portion of a lateral edge of the second base; **characterized by**:
a first clip (310) for maintaining at least a first loop of the first plurality of loops adjacent at least a first loop of the second plurality of loops, the first clip including a body (312) defining a cavity (311) and an extension (314) secured to the body, the extension being pivotable within the cavity to permit receipt of the first loops of the first and second plurality of loops within the cavity.

2. The system of claim 1, wherein the first clip (310) for maintaining the at least first loop of the first plurality of loops (104a) adjacent the at least first loop of the second plurality of loops (104b) includes a plurality of hook members formed on the at least first loop of the first plurality of loops and a plurality of loop members formed on the at least first loop of the second plurality of loops for engaging the hook members.

3. The system of any preceding claim, further include a clip applier (300) for applying the clip to the at least first loop of the first and second plurality of loops.

4. The system of any of claims 1 to 2, further include a stapling device (200) for forming a staple about the at least first loop of the first and second plurality of loops.

5. The system of any of claims 1 to 2, further include an applier for applying a wire about the at least first loop of the first and second plurality of loops.

6. The system of any preceding claim, further include a welding device for fusing at least the first loop of the first and second plurality of loops together.

7. The system of claim 1 wherein the clip body (312) has a substantially C-shape configuration defining the cavity, the body having a first end (312a) and a second end (312b) and a notch (313) formed in the second end for selectively receiving a free end (314a) of the extension (314).

## Patentansprüche

1. System zum laparoskopisch Verbinden von Patches (100), wobei das System umfasst:
ein erstes Patch (100a), das eine erste Basis (102a) und eine erste Vielzahl von Schlaufen (104a) aufweist, die sich entlang zumindest eines Abschnitts von einem seitlichen Rand der ersten Basis erstrecken;
ein zweites Patch (100b), das eine zweite Basis (102b) und eine zweite Vielzahl von Schlaufen (104b) aufweist, die sich entlang zumindest eines Abschnitts von einem seitlichen Rand der zweiten Basis erstrecken;
**gekennzeichnet durch**:
einen ersten Clip (310) zum Halten zumindest einer ersten Schlaufe von der ersten Vielzahl von Schlaufen benachbart zu zumindest einer ersten Schlaufe von der zweiten Vielzahl von Schlaufen, wobei der erste Clip einen Körper (312), der einen Hohlraum (311) definiert, und eine Erweiterung (314) aufweist, die an dem Körper gesichert ist, wobei die Erweiterung innerhalb des Hohlraums schwenkbar ist, um einen Empfang der ersten Schlaufen von der ersten und zweiten Vielzahl von Schlaufen innerhalb des Hohlraums zu ermöglichen.

2. System nach Anspruch 1, wobei der erste Clip (310) zum Halten der zumindest ersten Schlaufe von der ersten Vielzahl von Schlaufen (104a) benachbart zu der zumindest ersten Schlaufe von der zweiten Vielzahl von Schlaufen (104b) eine Vielzahl von Hakenelementen, die an der zumindest ersten Schlaufe von der ersten Vielzahl von Schlaufen ausgebildet sind, und eine Vielzahl von Schlaufenelementen aufweist, die an der zumindest ersten Schlaufe von der zweiten Vielzahl von Schlaufen für einen Eingriff mit den Hakenelementen ausgebildet sind.

3. System nach einem vorstehenden Anspruch, weiter einen Clip-Anbringer (300) aufweisen, um den Clip an der zumindest ersten Schlaufe von der ersten und zweiten Vielzahl von Schlaufen anzubringen.

4. System nach einem der Ansprüche 1 bis 2, weiter eine Klammervorrichtung (200) umfassen, um eine Klammer um die zumindest erste Schlaufe von der ersten und zweiten Vielzahl von Schlaufen zu formen.

5. System nach einem der Ansprüche 1 bis 2, weiter einen Anbringer umfassen, um einen Draht um die zumindest erste Schlaufe von der ersten und zweiten Vielzahl von Schlaufen anzubringen.

6. System nach einem vorstehenden Anspruch, weiter eine Schweißvorrichtung umfassen, um zumindest die erste Schlaufe von der ersten und zweiten Vielzahl von Schlaufen zu verschmelzen.

7. System nach Anspruch 1, wobei der Clip-Körper (312) eine im Wesentlichen C-förmige Konfiguration hat, die den Hohlraum definiert, wobei der Körper ein erstes Ende (312a) und ein zweites Ende (312b) und eine in dem zweiten Ende ausgebildete Kerbe (313) zum wahlweise Empfangen eines freien Endes (314a) der Erweiterung (314) aufweist.

## Revendications

1. Système de liaison de patchs (100) par voie laparoscopique, le système comprenant :
un premier patch (100a) comprenant une première base (102a) et une première pluralité de boucles (104a) s'étendant le long d'au moins une partie d'un bord latéral de la première base ;
un second patch (100b) comprenant une seconde base (102b) et une seconde pluralité de boucles (104b) s'étendant le long d'au moins une partie d'un bord latéral de la seconde base ; **caractérisé par** :
une première attache (310) pour maintenir au moins une première boucle de la première pluralité de boucles adjacente à au moins une première boucle de la seconde pluralité de boucles, la première attache comprenant un corps (312) définissant une cavité (311) et une extension (314) fixée au corps, l'extension pouvant pivoter dans la cavité pour permettre la réception des première boucles de la première et de la seconde pluralité de boucles dans la cavité.

2. Système selon la revendication 1, dans lequel la première attache (310) pour maintenir la au moins une première boucle de la première pluralité de boucles (104a) adjacente à la au moins une première boucle de la seconde pluralité de boucles (104b) comprend une pluralité d'éléments à crochet formés sur la au moins une première boucle de la première pluralité de boucles et une pluralité d'éléments de boucle formés sur la au moins une première boucle de la seconde pluralité de boucles pour s'engager sur les éléments à crochet.

3. Système selon l'une quelconque des revendications précédentes, comprenant en outre un applicateur d'attaches (300) pour appliquer l'attache à la au moins une première boucle de la première et de la seconde pluralité de boucles.

4. Système selon l'une quelconque des revendications 1 à 2, comprenant en outre une agrafeuse (200) pour former une agrafe autour de la au moins une première boucle de la première et de la seconde pluralité de boucles.

5. Système selon l'une quelconque des revendications 1 à 2, comprenant en outre un applicateur pour appliquer un fil métallique autour de la au moins une première boucle de la première et de la seconde pluralité de boucles.

6. Système selon l'une quelconque des revendications précédentes, comprenant en outre un dispositif de soudure pour fusionner au moins la première boucle de la première et de la seconde pluralité de boucles conjointement.

7. Système selon la revendication 1, dans lequel le corps d'attache (312) a une configuration sensiblement en forme de C définissant la cavité, le corps ayant une première extrémité (312a) et une seconde extrémité (312b) ainsi qu'une encoche (313) formée à la seconde extrémité pour recevoir sélectivement une extrémité libre (314a) de l'extension (314).
